Europäisches Patentamt

(19) European Patent Office

Office européen des brevets

(11) Veröffentlichungsnummer: **0 046 563**
**B1**

(12) **EUROPÄISCHE PATENTSCHRIFT**

(45) Veröffentlichungstag der Patentschrift:
10.04.85

(21) Anmeldenummer: 81106389.0

(22) Anmeldetag: 17.08.81

(51) Int. Cl.⁴: **G 01 N 21/76**, G 01 N 33/533

(54) Verfahren zur Durchführung analytischer Bestimmungen mittels der Chemilumineszenzmethode und Anwendung des Verfahrens für Immunoassays.

(30) Priorität: 22.08.80 GB 8027422

(43) Veröffentlichungstag der Anmeldung:
03.03.82 Patentblatt 82/9

(45) Bekanntmachung des Hinweises auf die Patenterteilung:
10.04.85 Patentblatt 85/15

(84) Benannte Vertragsstaaten:
CH DE FR IT LI SE

(56) Entgegenhaltungen:
US - A - 3 789 116
US - A - 4 067 959
US - A - 4 104 029

CHEMICAL ABSTRACTS, Band 86, Nr. 18, 2. Mai 1977,
Zusammenfassung Nr. 130321s, Seite 595 COLUMBUS
OHIO (US) & Khim. Vys. Energ. 1977, 11(1), 93-4 B.A.
RUSIN et al.: "Light emitters in chemiluminescence
reactions of luminol oxidation. Oxidation of luminol by
sodium hypochlorite and tetranitromethane"
CHEMICAL ABSTRACTS, Band 92, Nr. 14, 7. April 1980,
Zusammenfassung Nr. 117327c, Seite 420 COLUMBUS
OHIO (US) & Zh. Anal. Khim. 1979, 34(7), 1340-4 B.A.
RUSIN et al.: "Oxidation of
4-(dimethylamino)phthalhydrazide with hypochlorite
accompanied by chemiluminescence. Spectral

(73) Patentinhaber: **LABORATORIUM PROF. DR. RUDOLF
BERTHOLD, Calmbacher Strasse 22,
D-7547 Wildbad 1 (DE)**

(72) Erfinder: **Berthold, Fritz, Dr., Weissenburgstrasse 12a,
D-7530 Pforzheim (DE)**
Erfinder: **Kubisiak, Helmut, Talwiesenstrasse 43,
D-7547 Wildbad (DE)**

(74) Vertreter: **Dr. Elisabeth Jung Dr. Jürgen Schirdewahn Dr.
Gerhard Schmitt-Nilson Dr. Gerhard B. Hagen Dipl.-Ing.
Peter Hirsch, Clemensstrasse 30 Postfach 40 14 68,
D-8000 München 40 (DE)**

(56) Entgegenhaltungen: (Fortsetzung)
characteristics of hydrazide and reaction products"
THE JOURNAL OF PHYSICAL CHEMISTRY, Band 78, Nr.
17, 15. August 1974 R. NILSSON et al.: "Role of Singlet
Oxygen in Some Chemiluminescence and Enzyme
Oxidation Reactions", Seiten 1861-1863
NATURE, Band 279, 14. Juni 1979 J.S.A. SIMPSON et al.:
"A Stable Chemiluminscent-La-belled Antibody for
Immunologican Assays", Seiten 646-647
ANALYTICAL CHEMISTRY, Band 50, Nr. 8, Juli 1978 H.R.
SCHROEDER et al.: "Chemiluminescence Yields and
Detection Limits of Some Isoluminol Derivatives in
Various Oxidation Systems", Seiten 1114-1120

## Beschreibung

Die Chemilumineszenz ist ein physikalischer Vorgang, der auf einer chemischen Reaktion beruht und sich daher grundlegend von der Fluoreszenz unterscheidet, bei der das durch sichtbares oder UV-Licht angeregte Molekül unverändert bleibt und lediglich das absorbierte Licht wieder abstrahlt (emittiert), und zwar entweder bei gleicher oder längerer Wellenlänge.

Der Mechanismus der Chemilumineszenz beruht hingegen auf der Neubildung energiereicher, instabiler Zwischenprodukte, die unter Lichtaussendung zu einem Endprodukt zerfallen.

Analytische Verfahren unter Ausnutzung der Fluoreszenz sind an sich bekannt, doch hat sich dabei der Einfluss der Hintergrundfluoreszenz als ausserordentlich störend erwiesen, so dass man zu einem Kunstgriff Zuflucht nehmen musste, um diese Störfluoreszenz messtechnisch auszuschalten.

Gemäss der DE-A-2 628 158 wird dieses Ziel erreicht, indem man nur solche fluoreszierenden Stoffe als Markierungsmaterial verwendet, die bei passender Anregung und Zimmertemperatur ein schmales Fluoreszenzspektrum und gleichzeitig eine lange Zerfallszeit haben, und ausserdem durch einen entsprechenden Abtastmechanismus dafür sorgt, dass die Messung erst zu einem Zeitpunkt stattfindet, wo die Stör- beziehungsweise konkurrierende Fluoreszenz bereits abgeklungen ist, das heisst nach etwa 100 nano-Sekunden, gerechnet ab Strahlungsimpuls. Für diese Arbeitsweise eignen sich als Markierungsstoffe insbesondere Chelate seltener Erden und Pyrenbutyrate.

Diese Methode der DE-A-2 628 158 bietet jedoch den Nachteil, dass die eigentliche Messung immer erst nach der Abklingphase erfolgen kann und dass ausserdem eine relativ aufwendige Apparatur benötigt wird, um den Strahlungsimpuls so mit der Nachweiseinrichtung zu koppeln, dass die Messung nicht zu früh oder zu spät erfolgt.

Eine zusammenfassende Darstellung der bei den Fluoreszenz-Immunoassay-Verfahren auftretenden Schwierigkeiten findet sich in «Clinical Chemistry» (1979), SS. 353–361.

Vorschläge, die Chemilumineszenz für quantitative analytische Verfahren auszunützen, bedienen sich der Eigenschaft insbesondere der unter der Bezeichnung Luminol bekannten organischen Verbindung, in alkalischer Lösung mit Sauerstoff zu reagieren und dabei ein instabiles Zwischenprodukt zu bilden, das dann unter Lichtausstrahlung zerfällt. Hierzu wird auf die US-A-4 104 029 verwiesen, welche die Chemilumineszenzreaktion durch Zusatz von Alkalihypochlorit auslöst und Luminol als Markierungsmittel einsetzt.

Diese Methode hat jedoch bisher keinen Eingang in die analytische Praxis insbesondere bezüglich der Immunoassays finden können, wahrscheinlich vor allem deshalb, weil sich das Luminol bei der chemischen Modifizierung, die erforderlich ist, um diese Verbindung an Proteine vom Typ der Antigene und Antikörper ankoppelbar zu machen, derart im Molekülaufbau verändert, dass die für die Chemilumineszenz erforderliche Reaktion beeinträchtigt wird. Hierfür sprechen auch die Beobachtungen von Simpson und Mitarbeitern (vgl. «Nature», Vol. 279 (1979), SS. 646/647), dass diazotiertes Luminol nur noch knapp 1% der Strahlungsintensität von unverändertem Luminol aufweist.

Ausserdem wird im «Journal of Physical Chemistry» 78 (1974), SS. 1681–1683, ein System beschrieben, in dem Fluorescein durch chemische Prozesse zur Lumineszenz angeregt werden kann.

In dieser Literaturstelle wird das System $H_2O_2$ und NaOCl als Reaktionspartner für die Chemilumineszenzreaktion verwendet. Es wird jedoch über eine extrem hohe Konzentration des für einen Nachweis benötigten Fluoresceins berichtet. Die kleinste nachgewiesene Menge betrug $2 \times 10^{-4}$ Mol, so dass gegenüber den Erfordernissen beim Immunoassay mindestens sechs Grössenordnungen an Empfindlichkeit fehlen. Ausserdem wird beschrieben, dass die Lebensdauer der Lumineszenz in dem genannten System bei nur 2 «µs liegt, das heisst, die Photonenemission erfolgt blitzartig. Diese sehr schnelle Kinetik ist jedoch für eine empfindliche Messung völlig ungeeignet. Man verwendet als empfindlichste Messgeräte Photonenzähler, die aber wegen ihrer Totzeit von typisch 20 ns nur begrenzte maximale Impulsraten verarbeiten können. Blitzartige Lumineszenzerscheinungen können also quantitativ und empfindlich nicht gemessen werden.

Aufgabe der Erfindung war es daher, das Phänomen der Chemilumineszenz in für die Praxis brauchbarer Weise für Analysenmethoden und insbesondere Immunoassays anwendbar zu machen.

Um eine Anwendung der Chemilumineszenzmethode für einen Immunoassay zu ermöglichen, müssen mehrere Probleme gelöst werden:

1. Steigerung der Empfindlichkeit derart, dass mindestens $10^{-10}$ Mol des Markierungsmittels noch nachweisbar sind.

2. Veränderung der Reaktionskinetik derart, dass die Lichtemission nicht blitzartig, sondern zum Beispiel über mehrere Sekunden verteilt erfolgt.

3. Es wäre ferner wünschenswert, die Lumineszenz nicht durch Zugabe von zwei Reagenzien ($H_2O_2 + NaOCl$), sondern nur durch ein einziges Reagens einzuleiten. Dies würde einerseits die Messapparatur vereinfachen (es würde nur 1 statt 2 Dispensern benötigt); vor allem entsteht jedoch beim Mischen von zwei Reagenzien auch ohne Anwesenheit der nachzuweisenden lumineszierenden Substanz schon erhebliche Hintergrund-Lumineszenz. Diese ist bei dem System $H_2O_2$/ NaOCl sehr ausgeprägt und darüber hinaus nicht konstant, so dass man wieder die bei der Fluoreszenz bekannten Nachteile hätte.

Das erfindungsgemässe Verfahren zur Durchführung analytischer Bestimmungen mittels der Chemilumineszenzmethode und Messung der Lichtemission ist dadurch gekennzeichnet, dass man Fluoresceinisothiocyanat (FITC) als Markierungsmittel einsetzt und die Chemilumineszenzreaktion durch Zusatz einer wässrigen Lösung von Natriumhypochlorit auslöst.

Das Verfahren der Erfindung verwendet für die Chemilumineszenzreaktion ein überraschend wirksames neues System aus Markierungsmaterial und Oxidationsmitteln, das eine hohe Photonenausbeute ermöglicht und gleichzeitig bei der Kopplung mit beispielsweise Proteinen, Aminosäuren oder Polysacchariden keinen störenden Abfall der Reaktionsfähigkeit mit dem die Bildung der instabilen Zwischenprodukte induzierenden Oxidationsmittel zeigt. Ausserdem ist dieses System ausreichend stabil und sensitiv, so dass auch noch sehr geringe Konzentrationen des Markierungsmittels mit hoher Genauigkeit messbar sind.

Handelsübliche NaOCl-Lösungen enthalten etwa 150 bis 155 g Cl pro Liter. Im allgemeinen verwendet man für das erfindungsgemässe Verfahren wässrige verdünnte NaOCl-Lösungen, die beispielsweise 2 bis 100 g Cl/Liter enthalten.

Für Bestimmungen mit nicht an Protein gebundenem FITC eignen sich beispielsweise Hypochloritlösungen mit einem Gehalt von 2,5 g Cl pro Liter. Je nach dem Chlorgehalt der zu verdünnenden Ausgangslösung können daher die für die Chemilumineszenzreaktion angewendeten Hypochloritlösungen 0,01 bis 3% und zweckmässig 0,15 bis 0,6% (Gew/Vol) an NaOCl enthalten.

Für an Protein gebundenes FITC muss der Gehalt der Hypochloritlösung einen um den Faktor 10 höheren Chlorgehalt aufweisen, so dass man zweckmässig mit verdünnten Lösungen arbeitet, die etwa 25 g Cl pro Liter enthalten.

Die durch die Oxidationsreaktion induzierte Chemilumineszenz wird durch ein geeignetes Messgerät für Photonen quantitativ erfasst, ohne dass es eines besonderen Strahlungsimpulses bedarf, wie etwa bei der Fluoreszenzanalyse.

Das erfindungsgemässe Verfahren eignet sich sowohl für den Festphasenassay als auch für den Flüssigphasenassay.

Die betreffenden Targetsubstanzen, die durch die zur Chemilumineszenz anregbare Verbindung FITC markiert werden sollen, werden zuvor auf bekannte Weise von anderen Substanzen abgetrennt, zum Beispiel mittels Chromatographie oder Fixierung an Antikörper (vgl. hierzu «Proc. Soc. Experimental Biology», Bd. 113 (1963), SS. 394–397).

Beispielsweise wird der für eine bestimmte Targetsubstanz (Antigen) spezifische Antikörper an eine feste Matrixsubstanz, wie Celluloseacetat oder Polystyrol, gebunden. Sodann wird die gesuchte und analytisch zu bestimmende Targetsubstanz (Antigen) in gelöster Form mit dem an die Matrix gebundenen Antikörper inkubiert und dadurch an diese fixiert. Anschliessend wird an die fixierte Targetsubstanz das erfindungsgemässe Markierungsmittel FITC, das an einen Antikörper gekoppelt ist, gebunden. Dieses FITC wird durch Zugabe der wässrigen NaOCl-Lösung chemisch zur Lichtemission angeregt.

Die Ankoppelung des Markierungsmittels an den Antikörper oder das Antigen, je nachdem, ob man die direkte, die indirekte oder die antikomplementäre serologische Aktivitätsmethode wählt, erfolgt nach bekannten Methoden, wie sie bereits mit Erfolg für die Kopplung von Fluoresceinisothiocyanat verwendet worden sind (vgl. z.B. «Amer. J. Pathol.» 1.34 (1958), S. 1081 bzw. «Proc. Soc. Experimental Biol. Med.» 98 (1958), S. 898 ff.).

Beim direkten Lumineszenz-Immunoassay – beispielsweise zum Nachweis eines bestimmten Antigens im Blutserum – wird das Serum zunächst mit dem an die feste Phase gekoppelten spezifischen Antikörper inkubiert, wobei das gesuchte Antigen an die feste Phase gebunden wird. Die feste Phase wird sodann mit einem FITC-markierten Antikörper, der gegen das gesuchte Antigen gerichtet ist, inkubiert. Das erfindungsgemässe Markierungsmittel FITC wird so an das gesuchte Antigen gekoppelt.

Gemäss der bekannten «Sandwich»-Technik, beschrieben in «Proc. Soc. Ex. Biol.» 113 (1963), S. 349, wird ein festes Substrat mit daran gebundenem Antikörper mit der zu untersuchenden Probe inkubiert, wobei die Menge des an die feste Phase gebundenen Antikörpers über derjenigen liegen sollte, die für die Bindung des gesamten in der Probe enthaltenen Antigenmaterials erforderlich ist. Anschliessend inkubiert man die so mit dem Antigen beladene feste Phase mit einer Lösung, die den mit FITC markierten Antikörper enthält, so dass sich diese Antikörper an die Festphase mit daran gebundenen Antigenen binden. Durch Spülung werden anschliessend die nicht gebundenen FITC-markierten Antikörper entfernt. Sodann wird durch Zugabe der wässrigen NaOCl-Lösung die zu messende Chemilumineszenz induziert. Dieses sogenannte Sandwich-Verfahren lässt sich immer dann gut anwenden, wenn das betreffende Antigen mehr als eine Bindestelle für einen Antikörper aufweist, das heisst im allgemeinen für Antigene in Form grosser Moleküle.

Von besonderem Interesse ist das Verfahren gemäss der Erfindung in der virologisch-serologischen Diagnostik, zum Beispiel in der Hepatatis-A- und -B-Diagnostik, der Röteln-, CMV- und EBV-Serologie. Daneben besteht für die klinische Chemie grösstes Interesse an empfindlichen und spezifischen Testsystemen ohne Radioaktivität, zum Beispiel zur Schilddrüsendiagnostik (T3, T4), zur Diabetesdiagnostik (Insulin) usw.

Das erfindungsgemässe Verfahren wird anhand der nachstehenden Beispiele näher erläutert.

Beispiel 1

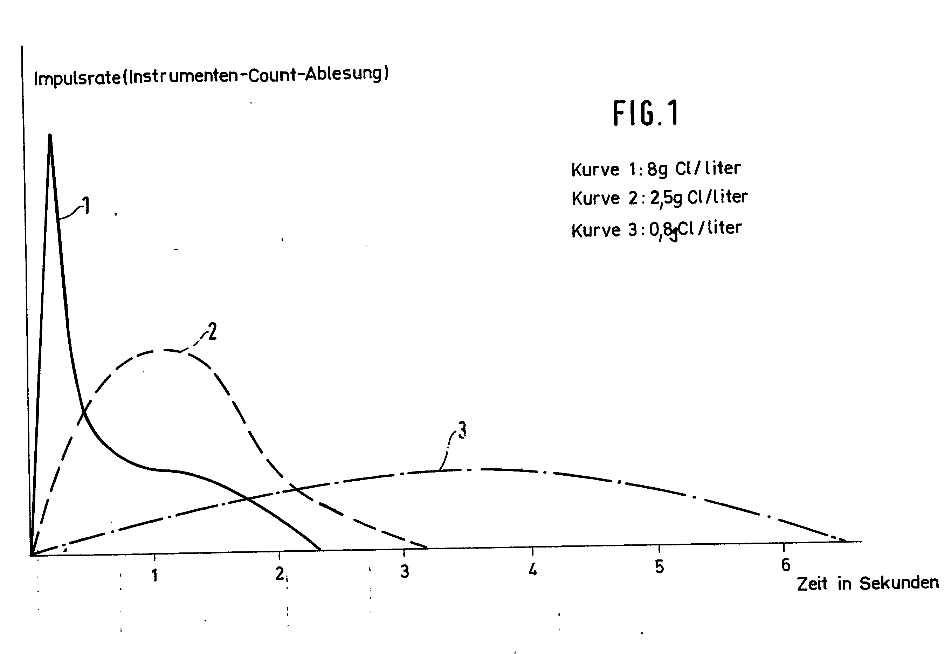

Im einzelnen wurden Messungen mit ungebundenem FITC zur Kinetik der Reaktion durchgeführt (siehe Fig. 1). Zunächst zeigte sich, dass der zeitliche Ablauf der Lichtemission, also die Kinetik, eindeutig über die NaOCl-Verdünnung eingestellt werden kann. Oberhalb einer Konzentration von ca. 8 g Cl pro Liter (Kurve 1) erhält man noch die bekannte blitzartige Lichtemission. Eine optimale Kinetik wurde im Bereich von ca. 2,5 g Cl pro Liter (Kurve 2) erhalten, während mit 0,8 g Cl pro Liter (Kurve 3) die Kinetik zwar noch langsamer wurde, die Empfindlichkeit aber schon beträchtlich zurückging.

Wurde anstelle des ungebundenen FITC proteingebundenes FITC verwendet, so ergab sich eine andere Kinetik als Funktion der Chlorkonzentration. Die Chlorkonzentration musste um einen Faktor 10 erhöht werden, um jeweils wieder die gleiche Kinetik wie bei ungebundenem FITC zu erzielen.

Die Messungen wurden dabei, wie folgt, durchgeführt:

Zunächst wurden 200 ng FITC, gelöst in 20 µl aqua dest., in ein Reagenzglas pipettiert. Dann wurde die Probe in die abgedunkelte Messposition vor den Photomultiplier gebracht (Messgerät BIOLUMAT LB 9500 der Firma Laboratorium Prof. Dr. Berthold, Wildbad). Dann wurden durch das Dispenser-System 100 µl NaOCl der angegebenen Verdünnung auf das FITC injiziert.

Die Kinetik wurde durch Registrierung über ein Ratemeter mit angeschlossenem Schreiber beobachtet. Das Ratemeter hat zwei Zeitkonstanten von 20 ms und 1 s, wobei automatisch die kurze Zeit bei schnellen Änderungen und die lange bei langsamen Änderungen eingeschaltet wird. Gleichzeitig wird die Impulszahl über eine Zeitdauer von vorzugsweise 10 s integriert.

Beispiel 2

In einer zweiten Versuchsreihe wurden Lösungen mit variierender FITC-Konzentration hergestellt. Dazu wurden variable Mengen FITC im Bereich von 1,25 bis 200 ng in jeweils 20 µl aqua dest. gelöst und anschliessend mit je 100 µl verdünnter NaOCl (2,5 g Cl pro Liter) beaufschlagt und ausgemessen.

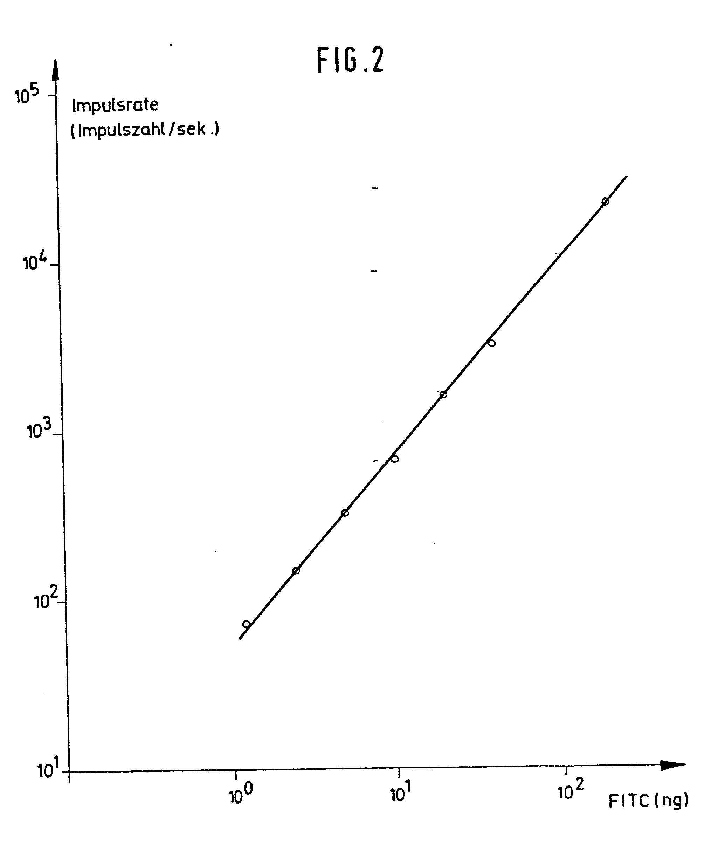

Das Ergebnis zeigt Fig. 2. Man erhält eine einwandfreie Linearität über den gesamten Bereich. Die Grenzen waren nur durch die derzeitig verfügbaren Messbedingungen gegeben. Zu grösseren Konzentrationen hin bestanden Probleme mit der Löslichkeit des verfügbaren FITC in Wasser, während nach unten hin mit Sicherheit ein weiterer Faktor 10 durch Erniedrigung des Gerätenulleffekts erreichbar ist, beispielsweise durch Kühlung des Photomultipliers.

Eine weitere Empfindlichkeitssteigerung um etwa zwei Grössenordnungen zeigte sich überraschenderweise, wenn das FITC nicht frei, sondern proteingebunden vorlag.

Beispiel 3

Das erfindungsgemässe Verfahren wird für den Nachweis des HBs-Antigens (nachstehend abgekürzt als HBs Ag) angewendet.

Es handelt sich hierbei um einen Festphasen-Assay. Anti-HBs vom Menschen wurde in üblicher Weise an die feste Phase gekoppelt (feste Phase: Polystyrol-Kügelchen oder Mikrotiterplatte). Nach einer anschliessenden Absättigung der festen Phase mit Kälberserum war diese gebrauchsfertig.

Die feste Phase wurde sodann mit der auf HBs Ag zu untersuchenden Serumprobe beziehungsweise einer Verdünnung derselben inkubiert. Je nach Testsystem betrugen die untersuchten Volumina 0,1 ml (Mikrotiterplatte) oder 0,2 ml Polystyrolkügelchen mit spezieller Bindungsfähigkeit für Antikörper. Für den Schnelltest betrug die Inkubationszeit 2 Stunden bei 37 °C, für den Standardtest 16 Stunden bei Zimmertemperatur. Anschliessend erfolgte gründliche Spülung mit PBS (phosphate buffered solution) in der üblichen Zusammensetzung:

| | |
|---|---|
| NaCl | 8,0 g |
| KCl | 0,2 g |
| $Na_2HPO_4 \cdot 12H_2O$ | 2,89 g |
| $KH_2PO_4$ | 0,2 g |
| Aqua bidest., | ad 1000 ml |

In der Serumprobe vorhandenes HBs Ag war nun an die feste Phase gebunden; alle nicht gebundenen Proteine wurden abgespült.

Anschliessend erfolgte die Zugabe des Tracers. Als Tracer wurde ein FITC-markiertes humanes Anti-HBs verwendet, das affinitätschromatographisch gewonnen worden war. Die feste Phase mit dem Tracer wurde 4 Stunden bei Zimmertemperatur inkubiert. Anschliessend erfolgte wiederum eine gründliche Spülung mit Phosphatpuffer. Der Tracer hatte nun an das an der festen Phase haftende HBs Ag gebunden.

Die zu untersuchende Serumprobe wurde unverdünnt sowie in unterschiedlichen Konzentrationen, verdünnt mit menschlichem Normalserum, eingesetzt. Als Verdünnungsmittel kann aber auch Phosphatpuffer verwendet werden, gegebenenfalls mit einem Zusatz von $NaN_3$ (0,1%ig), das bakterizid wirkt und in den Serumproben das Bakterienwachstum hemmt.

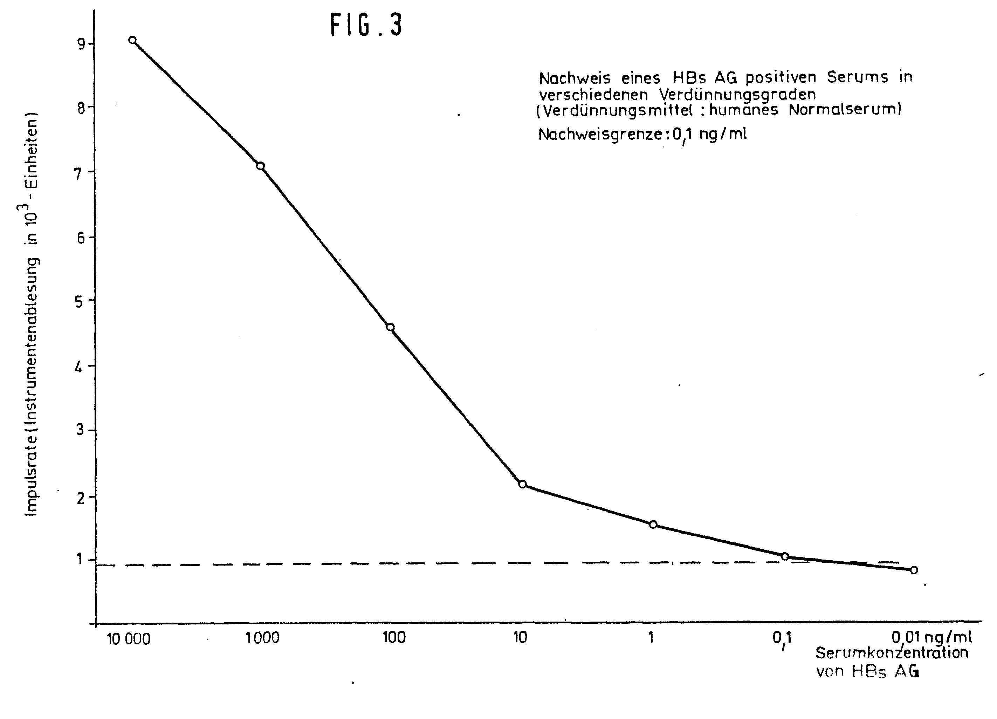

Die einzelnen Proben (Näpfchen einer Mikrotiterplatte oder Polystyrolkügelchen) wurden dann im Photonenzähler nach Zusatz von wässriger NaOCl-Lösung (25 g Cl pro Liter) untersucht. Die gemessenen Impulsraten wurden in Fig. 3 in Abhängigkeit von der HBs-Ag-Konzentration der untersuchten Serumprobe aufgetragen.

Die hierbei erhaltenen Ergebnisse sind in Fig. 3 wiedergegeben.

Die Nachweisgrenze für die Serumkonzentration des HBs Ag beträgt 0,1 ng/ml. Diese Kurve kann auch als Eichkurve für die Bestimmung unbekannter Konzentrationen an HBs Ag in Serumproben dienen.

Entscheidende Vorteile dieses neuen Verfahrens gemäss der Erfindung sind:

1. Es muss nur ein einziges Reagens (anstatt sonst zwei) zur Auslösung der Chemilumineszenzreaktion zugesetzt werden; somit kann auch das Messgerät wesentlich einfacher ausgerüstet sein, weil es nur einen und nicht zwei automatische Injektoren benötigt.

2. Der Nullwert ist jetzt nur noch durch das Gerät selbst bestimmt. Werden hingegen zwei Reagenzien zusätzlich zum FITC benötigt, so erzeugen diese meist schon in Abwesenheit von FITC eine beträchtliche Lumineszenz als Störfaktor.

3. Nur mit diesem System konnte eine einwandfreie Linearität der Anzeige zur FITC-Menge über zunächst einen Konzentrationsbereich von $10^0$ bis $2 \times 10^2$ festgestellt werden (Fig. 2), was einem Faktor von 200 entspricht.

4. Auch bei an Protein gebundenem FITC konnte ein breiter Konzentrationsbereich einwandfrei erfasst werden, wobei die Kurve bei sehr kleinen Konzentrationen deutlich flacher als im Konzentrationsbereich von 10 bis 1000 ng/ml verläuft.

**Patentansprüche**

1. Verfahren zur Durchführung analytischer Bestimmungen mittels der Chemilumineszenzmethode und Messung der Lichtemission, dadurch gekennzeichnet, dass man Fluoresceinisothiocyanat (FITC) als Markierungsmittel einsetzt und die Chemilumineszenzreaktion durch Zusatz einer wässrigen Lösung von Natriumhypochlorit auslöst.

2. Verfahren nach Anspruch 1, dadurch gekennzeichnet, dass man die Lichtemission mittels eines Gerätes misst, bei dem die Lichtquanten durch einen Photomultiplier nachgewiesen werden und die durch die Photonen ausgelösten Einzelimpulse gezählt werden.

3. Verfahren nach Anspruch 1 und 2, dadurch gekennzeichnet, dass man eine Natriumhypochloritlösung mit einem Gehalt von 2 bis 100 g Cl pro Liter verwendet.

4. Verfahren nach Anspruch 3, dadurch gekennzeichnet, dass man für die Bestimmung von nicht an Protein gebundenem FITC eine Natriumhypochloritlösung mit einem Gehalt von 2,5 g Cl pro Liter verwendet.

5. Verfahren nach Anspruch 3, dadurch gekennzeichnet, dass man für die Bestimmung von an Protein gebundenem FITC eine Natriumhypochloritlösung mit einem Gehalt an 25 g Cl pro Liter verwendet.

6. Verfahren nach Anspruch 1 bis 5, dadurch gekennzeichnet, dass man als Verdünnungsmittel destilliertes Wasser, Phosphatpuffer oder eine Mischung aus Phosphatpuffer und NaN₃-Lösung verwendet.

7. Anwendung des Verfahrens nach Anspruch 1 bis 6 zur Durchführung von Immunoassays.

**Claims**

1. A process for carrying out analytical determinations by means of chemiluminescence and measuring the emission of light, characterized in that fluoresceinisothiocyanate (FITC) is employed as a labelling agent and the chemilumiscence reaction is triggered by adding an aqueous solution of sodium hypochlorite.

2. A process according to claim 1, characterized in that the light emitted is measured by means of an instrument in which the quanta of light are detected by a photomultiplier and the individual pulses triggered off by the photons are counted.

3. A process according to claims 1 and 2, characterized in that a sodium hypochlorite solution containing from 2 to 100 g of Cl/litre is used.

4. A process according to claim 3, characterized in that a sodium hypochlorite solution containing 2,5 g of Cl/litre is used for the determination of FITC not bonded to protein.

5. A process according to claim 3, characterized in that a sodium hypochlorite solution containing 25 g of Cl/litre is used for the determination of protein-bonded FITC.

6. A process according to claims 1 to 5, characterized in that distilled water, phosphate buffer or a mixture of phosphate buffer and NaN₃ solution is used as diluent.

7. Use of the process according to anyone of claims 1 to 6 for carrying out immunoassay.

**Revendications**

1. Procédé pour l'exécution de déterminations analytiques par la méthode de chimiluminescence et mesure l'émission de lumière, procédé caractérisé en ce que l'on utilise comme marqueur l'isothiocyanate de fluorescéine (FITC) et on déclenche la réaction de chimiluminescence en ajoutant une solution aqueuse d'hypochlorite de sodium.

2. Procédé selon la revendication 1, caractérisé en ce que l'on mesure l'émission de lumière au moyen d'un appareil dans lequel les quanta de lumière sont décelés par un photomultiplicateur et les impulsions individuelles qui sont déclenchées par les photons sont comptées.

3. Procédé selon la revendication 1 ou 2, caractérisé en ce que l'on ajoute une solution d'hypochlorite de sodium ayant une teneur en chlore de 2 à 100 g par litre.

4. Procédé selon la revendication 3, caractérisé en ce que pour doser le FITC qui n'est pas lié à une protéine, on utilise une solution d'hypochlorite de sodium à 2,5 g de chlore par litre.

5. Procédé selon la revendication 3, caractérisé en ce que pour doser le FITC lié à la protéine on utilise une solution d'hypochlorite de sodium à 25 g de chlore par litre.

6. Procédé selon l'une quelconque des revendications 1 à 5, caractérisé en ce que l'on emploie comme diluant de l'eau distillée, un tampon au phosphate ou un mélange d'un tampon au phosphate et d'une solution d'azoture de sodium NaN₃.

7. L'application d'un procédé selon l'une quelconque des revendications 1 à 6 à l'exécution de dosages immunologiques.

Impulsrate(Instrumenten-Count-Ablesung)

FIG.1

Kurve 1: 8g Cl/liter
Kurve 2: 2,5g Cl/liter
Kurve 3: 0,8 Cl/liter

Zeit in Sekunden

0 046 563

# FIG.2

FIG. 3

Nachweis eines HBs AG positiven Serums in verschiedenen Verdünnungsgraden (Verdünnungsmittel : humanes Normalserum)

Nachweisgrenze: 0,1 ng/ml